# EUROPÄISCHE PATENTSCHRIFT

(11) **EP 3 105 131 B1**
(45) Veröffentlichungstag und Bekanntmachung des Hinweises auf die Patenterteilung: **30.01.2019**
(21) Anmeldenummer: 14812421.7
(22) Anmeldetag: 04.12.2014
(51) Int. Cl.: B65D 6/24, B65D 19/38, B65D 77/04, B01F 15/00

(54) **VERPACKUNG FÜR EINEN FLEXIBLEN BEHÄLTER UND TRANSPORTEINHEIT**
PACKAGING FOR A FLEXIBLE CONTAINER, AND A TRANSPORTATION UNIT
EMBALLAGE POUR RÉCIPIENT SOUPLE ET UNITÉ DE TRANSPORT

(30) Priorität: 13.02.2014 DE 102014101839
(43) Veröffentlichungstag der Anmeldung: 21.12.2016
(60) Teilanmeldung: 16187442.5
(73) Patentinhaber: Sartorius Stedim Biotech GmbH, 37079 Göttingen (DE)
(72) Erfinder: KRONENBERG, Diana, 13627 Berlin (DE)
(74) Vertreter: Prinz & Partner mbB
(86) Internationale Anmeldenummer: PCT/EP2014/076494
(87) Internationale Veröffentlichungsnummer: WO 2015/120927

(56) Entgegenhaltungen:
- EP-A1- 1 462 155
- WO-A1-2010/074953
- WO-A2-2013/171340
- DE-A1- 10 215 023
- DE-A1-102012 204 540
- DE-U1-202012 005 989
- GB-A- 2 189 773
- US-A- 3 556 886
- US-A- 4 290 300
- US-A- 5 897 012
- US-A- 6 029 839
- US-A1- 2005 272 146
- US-A1- 2009 260 551
- US-A1- 2011 310 696

## Beschreibung

Die Erfindung betrifft eine Verpackung für einen flexiblen Behälter, insbesondere für einen Einwegbeutel zur Verwendung in einem Bioreaktor. Die Erfindung betrifft ferner eine Transporteinheit mit einem Einwegbeutel, der in einer solchen Verpackung aufgenommen ist.

In Bioreaktoren werden bestimmte Mikroorganismen oder pflanzliche oder tierische Zellen unter möglichst optimalen Bedingungen kultiviert. Hierbei kommen oft vorsterilisierte Einwegbeutel zum Einsatz, die herkömmliche Kulturgefäße aus Glas oder Edelstahl ersetzen. Die Einwegbeutel sind bereits mit einer Vielzahl an vormontierten Schläuchen ausgestattet. Außerdem können vorsterilisierte Rührorgane in die Beutel integriert sein, die über einen Magnetkörper im Beutelinneren mit dem externen Rührmotor des Bioreaktors gekoppelt sind.

Beim Transport solcher Einwegbeutel in gewöhnlichen Verpackungen kann nicht ausgeschlossen werden, dass es durch den Kontakt des Beutels mit den Rührorganen oder anderen harten bzw. scharfkantigen Teilen an den Schläuchen, wie etwa Schlauchklemmen oder anderen Hilfsmitteln zur Fixierung der Schläuche im aufgerollten Zustand, zu einer Beschädigung des Beutels kommen kann. Dies liegt daran, dass der Beutel - bezogen auf seine vorgesehene Gebrauchsstellung - üblicherweise in horizontaler Lage zusammengelegt, verpackt und transportiert wird.

Die WO 2010/074 953 A1 zeigt einen Bioreaktor mit einem flexiblen Behälter, der als Einweg-Kunststoffbeutel ausgeführt sein kann. Der Bioreaktor weist ein Bodenteil zur Fixierung eines unteren Behälterabschnitts und ein Deckenteil zur Fixierung eines oberen Behälterabschnitts auf. Zwischen dem Bodenteil und dem Deckenteil sind Streben angeordnet, die einen Aufnahmeraum begrenzen, in dem der Behälter platziert ist.

Die US 5 307 928 zeigt eine Transportverpackung für Haushaltsgeräte, die eine obere Abdeckung und eine untere Unterstützungseinrichtung mit einer unteren Abdeckung und vier gefalzten Seitenelementen umfasst, in denen die Seitenkanten des Geräts aufgenommen sind. Während des Transports des Geräts werden die obere Abdeckung, die untere Abdeckung und die Seitenelemente durch Bänder am Gerät fixiert, um einen Schutz des Geräts vor Transportschäden zu gewährleisten.

Aus der WO 2013/171340 A2 ist eine Überverpackung für einen Bioreaktor-Beutel bekannt, welche aus einem Deckel, einem Boden und Seitenwänden besteht. Die Überverpackung weist ein Fach zur Aufnahme des Bioreaktor-Beutels auf, wobei das Volumen des Fachs durch eine Trenneinrichtung mit zwei im stumpfen Winkel aufeinander zulaufende Giebelwände begrenzt wird. Die Trenneinrichtung ist innerhalb der Überverpackung vertikal verschiebbar angeordnet, so dass das Volumen des Fachs variabel an verschiedene Volumina von Bioreaktor-Beuteln angepasst werden kann.

Eine Behältervorrichtung mit den Merkmalen des Oberbegriffs des Anspruchs 1 ist in der GB 2 189 773 A gezeigt. Die Mehrwegvorrichtung besteht im Wesentlichen aus einem zusammenschiebbaren Stützbehälter zum Abstützen eines darin aufgenommenen Beutels. Der Stützbehälter weist ein starres Oberteil und ein starres Unterteil sowie vier zusammenschiebbare Seitenteile aus einem zusammenhängenden flexiblen Material auf, die am Oberteil befestigt sind. Außerhalb der Seitenteile angeordnete faltbare Stützstreben, die am Oberteil und Unterteil befestigt sind, ermöglichen das Zusammenschieben der Seitenteile. Zum Entleeren des Beutels kann dessen oberer Abschnitt durch eine Zugangsöffnung im Oberteil gezogen und auf der Außenseite des Oberteils an schräg hervorstehenden Bolzen aufgespießt werden, die um die Zugangsöffnung herum angeordnet sind.

Die US 5 897 012 A zeigt ebenfalls eine Behältervorrichtung mit den Merkmalen des Oberbegriffs des Anspruchs 1 in Form eines Containers mit einem verformbaren Beutel, der an einem zusammenklappbaren Rahmen befestigt ist. Der Rahmen umfasst ein palettenartiges Bodenteil mit seitlichen Aussparungen, in die Zinken eines Gabelstaplers eingeführt werden können. Zwischen der unteren und der oberen Platte des palettenartigen Bodenteils ist zudem eine Ablaufleitung mit einem Ventil aufgenommen. Die Ablaufleitung ist über eine Öffnung in der oberen Platte zugänglich, die mittels einer Klappe verschließbar ist.

Aufgabe der Erfindung ist es, eine Verpackung bereitzustellen, die einen sicheren Transport und eine sichere Lagerung eines flexiblen Behälters ermöglicht, insbesondere unter Berücksichtigung der Schläuche, die am Behälter angebracht und in der Verpackung zu verstauen sind.

Gelöst wird diese Aufgabe durch eine Verpackung mit den Merkmalen des Anspruchs 1. Vorteilhafte und zweckmäßige Ausgestaltungen der erfindungsgemäßen Verpackung sind in den zugehörigen Unteransprüchen angegeben.

Die erfindungsgemäße Verpackung für einen flexiblen Behälter, insbesondere für einen Einwegbeutel zur Verwendung in einem Bioreaktor, umfasst ein Bodenteil, ein Deckenteil mit Mitteln zur Fixierung eines ersten Abschnitts des Behälters, einen Aufnahmeraum, der auf einer ersten Seite durch das Bodenteil und auf einer gegenüberliegenden zweiten Seite durch das Deckenteil begrenzt ist, und zwischen dem Bodenteil und dem Deckenteil angeordnete Streben, die einen definierten Abstand zwischen dem Bodenteil und dem Deckenteil festlegen. Das Bodenteil weist Mittel zur Fixierung eines dem ersten Abschnitt gegenüberliegenden zweiten Abschnitts des Behälters auf, sodass der fixierte flexible Behälter in einem Transportzustand dauerhaft in einem Entfaltungszustand gehalten werden kann. Gemäß der Erfindung sind das Bodenteil, das Deckenteil und die Streben aus demselben, gammasterilisierbaren Material, vorzugsweise Polyethylen (PE), gefertigt. Das Bodenteil und das Deckenteil weisen für jede Strebe gegenüberliegende Halterungen auf, in die die Streben zur Bildung eines Verpackungsgestells parallel zueinander eingesetzt sind.

Die gemäß der Erfindung vorgesehenen Streben dienen als Abstandshalter zwischen dem Bodenteil und dem Deckenteil und definieren damit die Höhe des Aufnahmeraums. Dadurch ist es bei Kenntnis der Abmessungen des zu transportierenden Behälters möglich, den Abstand zwischen dem Bodenteil und dem Deckenteil so groß zu wählen, dass der an diesen Teilen fixierte Behälter im Transportzustand dauerhaft in einem Entfaltungszustand gehalten wird, in dem ein Druck- oder Reibkontakt der flexiblen Behälterhülle mit innenliegenden Teilen, wie etwa einem Rührorgan, weitestgehend ausgeschlossen ist. Außerdem sorgen die Streben dafür, dass die Verpackung zu einer Art Gestell wird, in dem der Aufnahmeraum insgesamt ein so großes Volumen hat, dass die flexible Behälterhülle bei einem Kontakt mit einem harten oder scharfkantigen Teil praktisch immer eine Möglichkeit zum Ausweichen hat. Die Streben haben im Vergleich zu glatten Seitenwänden, wie sie etwa bei der Verpackung nach der WO 2013/171340 A2 vorgesehen sind, den Vorteil, dass sie leichter zu greifen sind. Das bedeutet, dass das Verpackungsgestell sowohl beim Verpacken als auch beim Auspacken leichter manipulierbar ist (Tragen, Kippen, Drehen etc.). Die erfindungsgemäße Verpackung kann in der gleichen vorteilhaften Weise auch als Verpackung zum Lagern des Behälters vor oder nach einem Transport dienen.

Die Begriffe "Bodenteil" und "Deckenteil" wurden im Hinblick auf die bevorzugte Anwendung der Erfindung gewählt, bei der die Orientierungen der Verpackung und des flexiblen Behälters eine wichtige Rolle spielen, worauf später noch genauer eingegangen wird. Die Begriffe sind jedoch nicht einschränkend zu verstehen. Bei anderen Anwendungen muss das Bodenteil nicht unbedingt unten und das Deckenteil nicht unbedingt oben angeordnet sein. Beispielsweise können die beiden Teile auch Seitenteile darstellen.

Bei einer nicht-erfindungsgemäßen Ausführungsform ist im Bodenteil und/oder im Deckenteil wenigstens ein Fach gebildet, das vom Aufnahmeraum über wenigstens eine Durchtrittsöffnung zugänglich ist. Ein solches Fach dient zur Aufnahme eines oder mehrerer Schläuche, die an den Behälter angeschlossen sind. Zum Verpacken wird der jeweilige Schlauch durch die Durchtrittsöffnung hindurch geführt, sodass der Großteil des Schlauchs in dem Fach zu liegen kommt. Durch die räumliche Trennung des Fachs vom Aufnahmeraum, in dem der Behälter aufgenommen ist, können Druck- oder Reibkontakte zwischen Behälterhülle und Schlauch ausgeschlossen werden. Außerdem kann durch die vorgegebene Führung und Platzierung eines Schlauchs in einem separaten Fach ein Knicken des Schlauchs vermieden werden. Dies kann durch weitere Maßnahmen unterstützt werden, wie etwa das Aufrollen und Fixieren des im Fach befindlichen Schlauchabschnitts. Im Vergleich zu einer konventionellen Verpackung gestaltet sich das Auspacken des Behälters einfacher, da jeder Schlauch an einer vorbestimmten Stelle vorgefunden wird. Wenn mehrere Fächer vorgesehen sind, in denen jeweils nur ein Schlauch untergebracht ist, wird dadurch ein Verheddern der Schläuche verhindert. Zu beachten ist hierbei, dass das wenigstens eine Fach - abgesehen von der wenigstens einen Durchtrittsöffnung - nicht ringsum geschlossen sein muss. Vielmehr sollte das Fach seitlich zugänglich sein, damit ein Schlauch leichter darin platziert oder daraus entnommen werden kann. Im Falle von mehreren Fächern kann ein Fach auch mit einem oder mehreren angrenzenden Fächern verbunden sein.

Vorzugsweise weist bei der nicht-erfindungsgemäßen Ausführungsform das Bodenteil und/oder das Deckenteil eine untere Platte und eine obere Platte auf, die durch ein oder mehrere Abstandselemente voneinander getrennt sind, wobei das wenigstens eine Fach durch die Platten und das bzw. die Abstandselemente definiert ist. Durch die beabstandete Anordnung von zwei Platten übereinander entsteht ein Freiraum, in dem das wenigstens eine Fach vorgesehen werden kann. Die Abstandselemente dienen gleichzeitig als seitliche Abgrenzungen des Fachs.

Bei einer weiteren nicht-erfindungsgemäßen Ausführungsform hat das Bodenteil und/oder das Deckenteil mehrere Durchtrittsöffnungen, die jeweils separat von einer Außenseite einer Platte des Bodenteils und/oder jeweils separat von einer Außenseite einer Platte des Deckenteils zugänglich sind. Die zusätzliche seitliche Zugänglichkeit der Durchtrittsöffnungen ermöglicht es, die Schläuche separat voneinander in verschiedene Durchtrittsöffnungen einzuschieben und separat voneinander zu verstauen. Dadurch wird ein Kabelsalat, genauer gesagt ein "Schlauchsalat" von mehreren ineinander verschlungenen Schläuchen vermieden, sodass das Auspacken und die Inbetriebnahme des Behälters mit den Schläuchen komfortabler durchführbar sind.

Bei den nicht-erfindungsgemäßen Ausführungsformen kann das Bodenteil und/oder das Deckenteil mit dem darin gebildeten Fach entweder einstückig ausgebildet oder aus mehreren Teilen zusammengesetzt und als vorgefertigte Baugruppe ausgeführt sein. In beiden Fällen vereinfacht sich das Verpacken, da das Bodenteil und/oder das Deckenteil ohne Vorarbeiten sofort als Ganzes verwendbar ist.

Im Hinblick auf die bevorzugte Anwendung der nicht-erfindungsgemäßen Verpackung weist eine Platte des Bodenteils oder des Deckenteils einen länglichen Schlitz auf, der sich von einer Außenseite der Platte nach innen erstreckt. In diesen Schlitz kann ein zentraler Abfluss- oder sonstiger Schlauch eines zu verpackenden Einwegbeutels seitlich eingeführt und zur Mitte gezogen werden. Der Schlitz dient also als Durchtrittsöffnung für den Schlauch, der dann auf der vom Aufnahmeraum abgewandten Seite der Platte abgelegt werden kann. Wenn der Schlauch auf dieser Seite zusammengerollt und fixiert wird, sodass der Schlauch nicht mehr zurück in den Aufnahmeraum gezogen werden kann, dient der Schlitz letztlich als Halterung für den Beutel.

Gemäß einer weiteren nicht-erfindungsgemäßen Ausführungsform weist ein Abstandselement auf dem Bodenteil bzw. Deckenteil quer zur Längsrichtung des Schlitzes eine Vertiefung auf. Der Schlauch kann somit durch die Vertiefung auf die vom Schlitz abgewandte Seite des Abstandselements geführt und dort abgelegt werden, wo er sicher vor Kontakten mit anderen Teilen des Behälters ist.

Um zu verhindern, dass sich der Schlauch vom Schlitzende wieder nach außen bewegt, kann bei einer nicht-erfindungsgemäßen Ausführungsform in der Platte vor dem inneren Schlitzende eine sich quer über den Schlitz erstreckende Ausnehmung gebildet sein, in die ein separates Rückhalteteil einsetzbar ist. Das Rückhalteelement stellt eine Sperre für den Schlauch dar und stellt einen definierten, knickfreien Verlauf des Schlauchs sicher.

Zur Verankerung der Streben weisen sowohl das Bodenteil als auch das Deckenteil einander gegenüberliegende Halterungen für die Streben auf.

Wiederum im Hinblick auf die bevorzugte Anwendung ist bei einer nicht-erfindungsgemäßen Ausführungsform eine zentrale Aufnahme im Bodenteil oder im Deckenteil für einen Magnetkörper eines in dem Behälter angeordneten Mischorgans vorteilhaft, der üblicherweise an einem Endabschnitt des Behälters angeordnet ist.

Bei einer nicht-erfindungsgemäßen Weiterbildung kann bei der Verpackung wenigstens ein separates Halterungsteil zur Fixierung von einem oder mehreren Schläuchen vorgesehen sein, die sich vom flexiblen Behälter erstrecken. Solche Halterungsteile stellen sicher, dass es nicht zu einem direkten Kontakt zwischen harten oder scharfkantigen Anschlüssen, Verbindungsstücken oder sonstigen Teilen der Schläuche und dem flexiblen Behälter kommt. Auch ein unerwünschtes Knicken der Schlauchabschnitte, die in den Behälter münden, wird durch die Fixierung mithilfe der Halterungsteile vermieden. Die Halterungsteile können hinsichtlich Form, Material etc. flexibel an die jeweiligen Schlauchdesigns angepasst werden, um einen optimalen Schutz vor Beschädigungen des Behälters und der Schläuche zu gewährleisten.

Das Halterungsteil kann bei einer nicht-erfindungsgemäßen Ausführungsform beispielsweise so gestaltet sein, dass wenigstens eine Leitungsaufnahme, in der wenigstens ein Schlauchabschnitt fixierbar ist, über eine Passage zugänglich ist. Der Schlauchabschnitt kann somit auf einfache Weise in die Leitungsaufnahme eingeführt und dort eingeklemmt und/oder durch Reibung gehalten werden.

Die Erfindung schafft auch eine Transporteinheit mit einem Einwegbeutel zur Verwendung in einem Bioreaktor, wobei der Einwegbeutel in einer Verpackung der oben beschriebenen Art aufgenommen ist.

Bezüglich der Vorteile dieser Transporteinheit wird auf die obigen Ausführungen zu den Vorteilen der erfindungsgemäßen Verpackung verwiesen.

Gemäß der bevorzugten Anwendung sind bei einer nicht-erfindungsgemäßen Ausführungsform an einem bezüglich der vorgesehenen Gebrauchsstellung des Behälters oberen Abschnitt des Behälters mehrere Schläuche angebracht, die durch eine oder mehrere Durchtrittsöffnungen des Bodenteils oder des Deckenteils in das bzw. die Fächer geführt sind. Bei einem Einwegbeutel zur Verwendung in einem Bioreaktor sind die benötigten Schläuche bereits an entsprechenden Anschlüsse der Beutelhülle montiert und werden als Einheit ausgeliefert. Die Mehrzahl der Schläuche mündet dabei in den oberen Abschnitt des Beutels, sodass gerade hier ein Bedarf besteht, die Schläuche geordnet und sicher zu verstauen.

Besonders bevorzugt sind bei einer nicht-erfindungsgemäßen Ausführungsform die eine oder mehreren Durchtrittsöffnungen des Bodenteils und/oder des Deckenteils jeweils separat von einer Außenseite einer Platte des Bodenteils und/oder jeweils separat von einer Außenseite einer Platte des Deckenteils zugänglich. Wie bereits erläutert, ermöglicht die zusätzliche seitliche Zugänglichkeit der Durchtrittsöffnungen, die Schläuche separat voneinander in verschiedene Durchtrittsöffnungen einzuschieben und separat voneinander zu verstauen, wodurch ein Verheddern von mehreren Schläuchen vermieden wird.

Vorzugsweise steht bei einer nicht-erfindungsgemäßen Ausführungsform jede Durchtrittsöffnung in Verbindung mit einem separaten Fach, sodass eine eindeutige Zuordnung der Durchtrittsöffnungen zu den Fächern gewährleistet ist.

Es erweist sich als vorteilhaft, bei einer nicht-erfindungsgemäßen Ausführungsform den Behälter bezüglich seiner vorgesehenen Gebrauchsstellung mit einem oberen Abschnitt am Bodenteil und mit einem unteren Abschnitt am Deckenteil zu fixieren (upside down). Wie oben erwähnt, befindet sich die Mehrzahl der Schläuche am oberen Abschnitt des Behälters, ebenso wie z. B. ein Magnetkörper zur Kopplung des Antriebs für die Rührorgane. Wenn der Behälter dann verkehrt herum verpackt und transportiert wird, liegt der Schwerpunkt des Behälters unten, und es besteht eine erheblich geringere Gefahr des Kippens der Transporteinheit.

Beim Verpacken eines Einwegbeutels, bei dem an einem bezüglich der vorgesehenen Gebrauchsstellung unteren Abschnitt (nur) ein zentraler Schlauch angebracht ist, ist es bei einer nicht-erfindungsgemäßen Ausführungsform vorteilhaft, diesen durch eine Durchtrittsöffnung des Deckenteils in Form eines Schlitzes in das wenigstens eine Fach zu führen.

Ein knickfreier Verlauf eines solchen Schlauchs wird bei einer nicht-erfindungsgemäßen Ausführungsform dadurch verhindert, dass das Bodenteil oder das Deckenteil wenigstens zwei Stützelemente aufweist, auf denen der Schlauch abgelegt ist.

Bei einer erfindungsgemäßen Ausführungsform weist der verpackte Behälter eine Welle mit einem auf einem Ende der Welle gelagerten Magnetkörper eines Mischorgans auf, der in eine Aufnahme des Bodenteils oder des Deckenteils eingesetzt ist. Diese Aufnahme bietet einen zusätzlichen Schutz davor, dass der Magnetkörper in unerwünschter Weise mit der Behälterhülle in Kontakt kommt.

Insbesondere für einen Behälter, der keine Welle oder sonstige Bauteile aufweist, die die Höhe des Beutels vorgeben, ist es bei einer nicht-erfindungsgemäßen Ausführungsform sinnvoll, die Anordnung und die Längen der Streben so auf die Ausdehnung des aufgenommenen Behälters in einer ersten Richtung zwischen dem Bodenteil und dem Deckenteil abzustimmen, dass der Behälter in den zur ersten Richtung senkrechten Richtungen nicht oder nur geringfügig zwischen den Streben hervorragt. Je weniger Spiel der Behälter in diesen Richtungen hat, desto geringer ist das Risiko einer Beschädigung.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine perspektivische Ansicht einer Verpackung gemäß einer ersten Ausführungsform der Erfindung;
- Figur 2 eine perspektivische Ansicht der Verpackung aus Figur 1 mit einem Einwegbeutel für einen Bioreaktor als erfindungsgemäße Transporteinheit;
- Figur 3 eine perspektivische Detailansicht des Deckenteils der Verpackung aus Figur 1;
- Figur 4 ein Halterungsteil einer erfindungsgemäßen Verpackung zur Fixierung von Schläuchen mit Anschlüssen;
- Figur 5 das Halterungsteil aus Figur 4 mit fixierten Schläuchen;
- Figur 6 eine perspektivische Ansicht einer Verpackung gemäß einer zweiten Ausführungsform der Erfindung; und
- Figur 7 eine perspektivische Ansicht der Verpackung aus Figur 6 mit einem Einwegbeutel für einen Bioreaktor als erfindungsgemäße Transporteinheit.

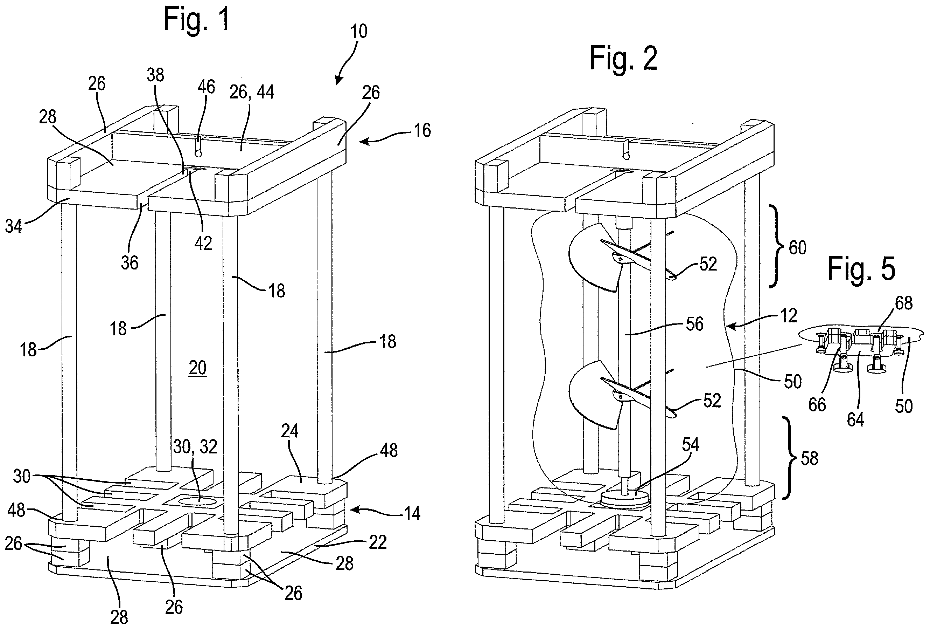

In den Figuren 1 und 2 ist eine erste Ausführungsform einer Verpackung 10 für einen flexiblen Behälter 12, genauer gesagt einen Einwegbeutel (siehe Figur 2), dargestellt, der zur Verwendung in einem Bioreaktor vorgesehen ist. Die Verpackung 10 umfasst ein Bodenteil 14, ein Deckenteil 16 und mehrere säulenartige Streben 18, die als Abstandshalter zwischen dem Bodenteil 14 und dem Deckenteil 16 dienen. Der durch das Bodenteil 14, das Deckenteil 16 und die Streben 18 definierte Raum bildet einen Aufnahmeraum 20 für den zu verpackenden Behälter 12.

Der Einfachheit halber wird nachfolgend entsprechend der bevorzugten Benutzung der Verpackung 10, auf die später noch eingegangen wird, die Längsrichtung der Streben 18 als vertikale Richtung und die dazu senkrechten Richtungen, in denen sich das Bodenteil 14 und das Deckenteil 16 erstrecken, als horizontale Richtungen bezeichnet, wobei diese Bezeichnungen nicht einschränkend zu verstehen sind.

Das Bodenteil 14 besteht im Wesentlichen aus einem Schaumstoff und weist eine untere Bodenplatte 22 und eine obere Bodenplatte 24 auf, die durch Abstandselemente 26 voneinander getrennt sind. Zwischen der unteren Bodenplatte 22 und der oberen Bodenplatte 24 befindet sich somit ein Freiraum, der durch die Abstandselemente 26 in mehrere Fächer 28 unterteilt ist. Ein Fach 28 kann seitlich vollständig geschlossen oder mit einem oder mehreren angrenzenden Fächern 28 verbunden sein. Wenigstens einige der Fächer 28 sind in horizontaler Richtung von außen zugänglich.

Die obere Bodenplatte 24 hat mehrere vertikale Durchtrittsöffnungen 30, durch die die Fächer 28 auch in vertikaler Richtung zugänglich sind. Die Durchtrittsöffnungen 30 können je nach Anforderung verschiedene Formen und Dimensionen haben. Im dargestellten Ausführungsbeispiel hat die obere Bodenplatte 24 mehrere rechteckige Öffnungen, die außenseitig nicht geschlossen sind, und eine zentrale Aufnahme 32.

Das Bodenteil 14 kann einstückig oder aus mehreren Einzelteilen zusammengesetzt sein. In letzterem Fall sind die Einzelteile so miteinander verklebt, verspannt, verklemmt, verschweißt oder auf sonstige Weise verbunden, dass das Bodenteil 14 eine vorgefertigte Baugruppe bildet und als Ganzes gelagert und bewegt werden kann.

Analog zum Bodenteil 14 ist das Deckenteil 16 entweder einstückig oder als vorgefertigte Baugruppe ausgebildet und besteht im Wesentlichen aus Schaumstoff. Auf einer Deckenplatte 34 des Deckenteils 16 sind auf der vom Bodenteil 14 abgewandten Seite mehrere Abstandselemente 26 angeordnet, jedoch keine weitere Platte zur vertikalen Begrenzung der durch die Abstandselemente angedeuteten Fächer. Eine solche zusätzliche Platte kann aber optional vorgesehen sein.

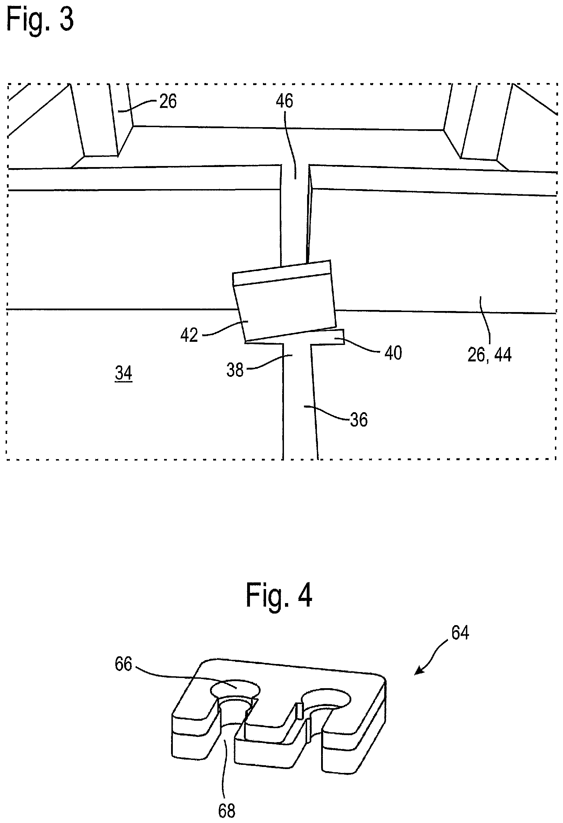

Im dargestellten Ausführungsbeispiel hat die Deckenplatte 34 als vertikale Durchtrittsöffnung 30 einen länglichen Schlitz 36, der sich von einer Außenseite bis etwa zur Mitte der Deckenplatte 34 erstreckt. Kurz vor dem etwas vergrößerten mittigen Ende 38 des Schlitzes 36 ist eine quer zur Längsrichtung des Schlitzes 36 verlaufende Ausnehmung 40 in der Deckenplatte 34 gebildet, die von einem separaten Rückhalteteil 42 ausgefüllt werden kann (siehe Figur 3). Auf der gegenüberliegenden Seite des Schlitzendes 38 erstreckt sich ebenfalls quer zur Längsrichtung des Schlitzes 36 ein längliches Abstandselement 44. In der gedachten Verlängerung des Schlitzes 36 weist dieses Abstandselement 44 auf seiner von der Deckenplatte 34 abgewandten freien Seite eine Vertiefung 46 auf.

Das Bodenteil 14, das Deckenteil 16 und die Streben 18 sind aus demselben, gammasterilisierbaren Material, vorzugsweise Polyethylen (PE), gefertigt.

Das Bodenteil 14 und das Deckenteil 16 weisen für jede Strebe 18 gegenüberliegende Halterungen 48 auf. Wenn die Streben 18 parallel zueinander in die Halterungen 48 eingesetzt sind, entsteht ein robustes Verpackungsgestell, dessen bevorzugte Verwendung nachfolgend genauer erläutert wird.

Ein weiteres, optionales Bestandteil der Verpackung 10 ist das in Figur 4 gezeigte Halterungsteil 64, von dem auch mehrere vorgesehen sein können. Das Halterungsteil 64 ist kammartig geformt und hat mehrere Leitungsaufnahmen 66, die über verengte Passagen 68 zugänglich sind.

Der zu verpackende Behälter 12 ist hier ein vorsterilisierter Einwegbeutel mit einer flexiblen Hülle 50. Der Behälter 12 enthält ein integriertes Rührwerk, das mehrere Rührorgane 52 und einen Magnetkörper 54 aufweist. Die Rührorgane 52 und der Magnetkörper 54 sind auf einer zentralen Welle 56 gelagert. Außerdem sind an den Behälter 12 mehrere (in den Figuren 1 und 2 nicht gezeigte) Fluidleitungen (Schläuche) angeschlossen. Gemäß der durch die Verwendung des Behälters 12 in einem Bioreaktor vorgesehenen Orientierung im Gebrauchszustand ist die Mehrzahl dieser Schläuche am oberen Abschnitt 58 des Behälters 12 vorgesehen. Am unteren Abschnitt 60 des Behälters 12 ist ein zentraler Abflussschlauch angeschlossen. Zumindest an einigen der Schläuche befinden sich Verbindungsstücke, Fixierklemmen oder andere harte bzw. scharfkantige Elemente.

Zum Verpacken wird der Behälter 12 zunächst mit seinem oberen Ende am Bodenteil 14 der Verpackung 10 fixiert, wobei der am oberen Wellenende angeordnete Magnetkörper 54 in die zentrale Aufnahme 32 der oberen Bodenplatte 24 eingesetzt wird. Die am oberen Abschnitt 58 des Behälters 12 angebrachten Schläuche werden durch die jeweils nächstliegende Durchtrittsöffnung 30 hindurchgeführt und im darunterliegenden Fach 28 aufgenommen. Die in den Fächern 28 befindlichen Schlauchabschnitte können z. B. durch Klemmen im aufgerollten Zustand fixiert und/oder durch Einwickeln oder Eintüten geschützt werden.

Anschließend werden die Streben 18 in die Halterungen 48 des Bodenteils 14 eingesetzt, und der Behälter 12 wird am zentralen Abflussschlauch nach oben gezogen. Zur Fixierung des unteren Behälterabschnitts 60 am Deckenteil 16 wird der Abflussschlauch durch den Schlitz 36 in der Deckenplatte 34 zur Mitte gezogen und über die Vertiefung 46 im Abstandselement 44 geführt, sodass der verbleibende Teil des Abflussschlauches auf der vom Schlitz 36 abgewandten Seite auf der Deckenplatte 34 zu liegen kommt. Auch dieser Schlauchteil kann im aufgerollten Zustand fixiert und durch weitere Maßnahmen geschützt werden.

Damit der Abflussschlauch einen definierten, knickfreien Verlauf vom Aufnahmeraum 20 über das Abstandselement 44 nimmt, wird das Rückhalteteil 42 in die Ausnehmung 40 eingesetzt, nachdem der Abflussschlauch bis zum Schlitzende 38 gezogen wurde. Durch das Rückhalteteil 42 wird verhindert, dass der Abflussschlauch nach außen verschoben werden kann. (Beim späteren Auspacken des Behälters 12 wird das Rückhalteteil 42 wieder aus der Ausnehmung 40 entfernt.)

Das Deckenteil 16 wird vor, während oder nach dem Fixieren des unteren Behälterabschnitts 60 am Deckenteil 16 auf die Streben 18 aufgesetzt.

Gemäß dem in Figur 2 gezeigten bevorzugten Transportzustand ist der Behälter 12 nun bezüglich seines späteren Gebrauchszustands verkehrt herum (upside down), d. h. mit dem oberen Abschnitt 58 unten und dem unteren Abschnitt 60 oben, in der Verpackung 10 aufgenommen.

Der durch die Länge der Streben 18 definierte Abstand zwischen dem Bodenteil 14 und dem Deckenteil 16 ist so gewählt, dass kein oder nur ein vernachlässigbarer Teil des fixierten Behälters 12 seitlich zwischen den Streben 18 aus dem Verpackungsgestell herausragen kann.

Zu einem beliebigen Zeitpunkt, bevor der Behälter endgültig transportfertig gemacht wird, können noch die separaten Halterungsteile 64 eingesetzt werden, wie in Figur 5 gezeigt. Die Halterungsteile 64 dienen dazu, sich von der flexiblen Hülle 50 des Behälters 12 erstreckende Schläuche zu fixieren. Dazu werden die Schläuche nahe der Hülle 50 durch die Passagen 68 in die Leitungsaufnahmen 66 der Halterungsteile 64 gedrückt. Dadurch wird erreicht, dass diejenigen Abschnitte der Schläuche, die in den Behälter 12 münden, so fixiert sind, dass sie sich mehr oder weniger senkrecht von der Hülle 50 nach außen erstrecken. Diese Fixierung sorgt dafür, dass harte oder scharfkantige Anschlüsse, Verbindungsstücke oder sonstige Teile der Schläuche von der flexiblen Hülle 50 ferngehalten werden, um Beschädigungen an der Hülle 50 selbst, aber auch an den Schläuchen und deren Anschlüssen, Verbindungsstücken etc. zu verhindern.

Um den Behälter 12 endgültig transportfertig zu machen, wird das Verpackungsgestell noch mit einem oder zwei Plastiksäcken oder dergleichen überzogen, der/die zugeschweißt oder mit Klebeband verschlossen werden, bevor die ganze Einheit dann in einen Versandkarton gestellt und mit Umreifungsbändern oder Packband gesichert wird.

Die in den Figuren 1 bis 5 gezeigte Verpackung 10 ist geeignet für große flexible Behälter 12 mit einem Füllvolumen in der Größenordnung von 500 Liter, 1.000 Liter, 2.000 Liter oder mehr.

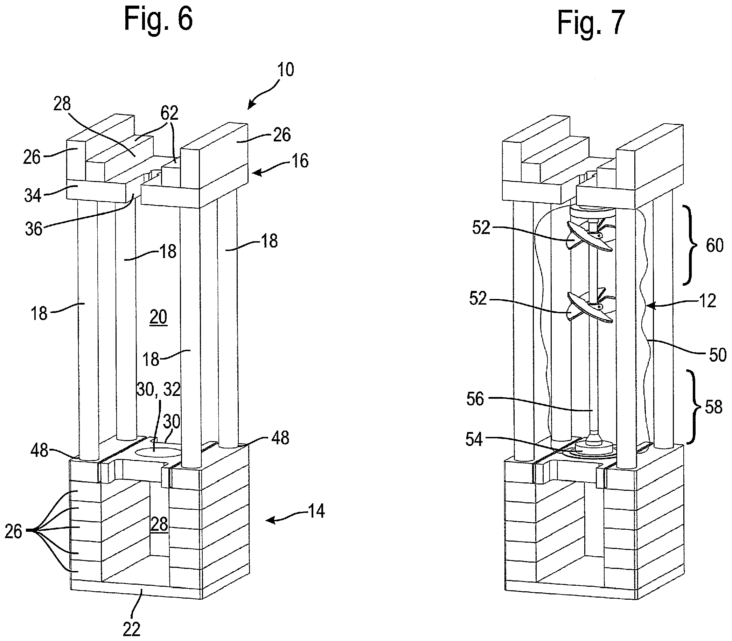

In den Figuren 6 und 7 ist eine zweite Ausführungsform der Verpackung 10 gezeigt, die für kleinere Behälter 12 mit einem Füllvolumen in der Größenordnung von 50 Liter oder 200 Liter geeignet ist. Die zweite Ausführungsform hat im Wesentlichen den gleichen Aufbau wie die erste Ausführungsform und unterscheidet sich nur in den Abmessungen der einzelnen Elemente und in der konkreten Formgestaltung des Bodenteils 14 und des Deckenteils 16. So ist bei der zweiten Ausführungsform nur ein einziges Fach 28 im Bodenteil gebildet, das jedoch im Vergleich zur ersten Ausführungsform eine größere vertikale Höhe hat. Ein quer über die Deckenplatte 34 verlaufendes Abstandselement und ein separates Rückhalteelement sind im Deckenteil 16 nicht vorgesehen. Dafür weist das Deckenteil 16 hier zwei Stützelemente 62 auf, die niedriger als die äußeren Abstandselemente 26 des Deckenteils 16 sind. Auf diesen Stützelementen 62 kommt der zusammengerollte Abflussschlauch zu liegen. Somit sorgen hier die Stützelemente 62 dafür, dass ein Knicken des Abflussschlauchs vermieden wird.

Beiden Ausführungsformen ist gemeinsam, dass die Bodenteile 14 und die Deckenteile 16 jeweils eine rechteckige, besonders bevorzugt eine quadratische Grundform haben. Diese Form ist aber nicht zwingend erforderlich, ebenso wie die Anzahl der Streben 18, d. h. es können je nach Anforderung auch weniger (insbesondere drei) oder mehr als vier Streben 18 vorgesehen sein, wobei immer eine Anordnung im Außenbereich bevorzugt ist. Das Bodenteil 14 und/oder das Deckenteil 16 können auch mehrere Zwischenböden mit Durchtrittsöffnungen 30 aufweisen, sodass die Fächer 28 für die Schläuche etc. auch übereinander angeordnet sein können.

Selbstverständlich können auch bei der zweiten Ausführungsform ein oder mehrere Halterungsteile 64 vorgesehen sein.

Grundsätzlich können Merkmale der verschiedenen Ausführungsformen in geeigneter Weise miteinander kombiniert werden, um weitere Ausführungsformen im Rahmen der Erfindung zu erhalten.

Die beschriebenen Ausführungsformen betreffen jeweils eine Verpackung 10 für einen speziellen Einwegbeutel, der zur Verwendung in einem Bioreaktor vorgesehen ist. Die Erfindung ist jedoch nicht auf diese Anwendung beschränkt. Vielmehr kann die erfindungsgemäße Verpackung 10 auch für andere flexible Behälter oder ähnliche Gegenstände verwendet werden.

### Bezuaszeichenliste

- 10: Verpackung
- 12: Behälter
- 14: Bodenteil
- 16: Deckenteil
- 18: Strebe
- 20: Aufnahmeraum
- 22: untere Bodenplatte
- 24: obere Bodenplatte
- 26: Abstandselement
- 28: Fach
- 30: Durchtrittsöffnung
- 32: Aufnahme
- 34: Deckenplatte
- 36: Schlitz
- 38: Schlitzende
- 40: Ausnehmung
- 42: Rückhalteteil
- 44: Abstandselement
- 46: Vertiefung
- 48: Halterung
- 50: Hülle
- 52: Rührorgan
- 54: Magnetkörper
- 56: Welle
- 58: oberer Behälterabschnitt
- 60: unterer Behälterabschnitt
- 62: Stützelement
- 64: Halterungsteil
- 66: Leitungsaufnahme
- 68: Passage

## Patentansprüche

1. Verpackung (10) für einen flexiblen Behälter (12), insbesondere für einen Einwegbeutel zur Verwendung in einem Bioreaktor, mit
einem Bodenteil (14),
einem Deckenteil (16) mit Mitteln zur Fixierung eines ersten Abschnitts (60) des Behälters (12),
einem Aufnahmeraum (20), der auf einer ersten Seite durch das Bodenteil (14) und auf einer gegenüberliegenden zweiten Seite durch das Deckenteil (16) begrenzt ist, und
zwischen dem Bodenteil (14) und dem Deckenteil (16) angeordneten Streben (18), die einen definierten Abstand zwischen dem Bodenteil (14) und dem Deckenteil (16) festlegen,
wobei das Bodenteil (14) Mittel zur Fixierung eines dem ersten Abschnitt (58) gegenüberliegenden zweiten Abschnitts (58) des Behälters (12) aufweist, sodass der fixierte flexible Behälter (12) in einem Transportzustand dauerhaft in einem Entfaltungszustand gehalten werden kann,
**dadurch gekennzeichnet,**
**dass** das Bodenteil (14), das Deckenteil (16) und die Streben (18) aus demselben, gammasterilisierbaren Material, vorzugsweise Polyethylen (PE), gefertigt sind, und
**dass** das Bodenteil (14) und das Deckenteil (16) für jede Strebe (18) gegenüberliegende Halterungen (48) aufweisen, in die die Streben (18) zur Bildung eines Verpackungsgestells parallel zueinander eingesetzt sind.

2. Transporteinheit, mit einem Einwegbeutel (12) zur Verwendung in einem Bioreaktor, **dadurch gekennzeichnet, dass** der Einwegbeutel (12) in einer Verpackung (10) nach Anspruch 1 aufgenommen ist, wobei der erste Abschnitt (60) des Einwegbeutels (12) am Deckenteil (16) und der gegenüberliegende zweite Abschnitt (58) des Einwegbeutels (12) am Bodenteil (14) fixiert ist, sodass der fixierte Einwegbeutel (12) in einem Transportzustand dauerhaft in einem Entfaltungszustand gehalten wird.

3. Transporteinheit nach Anspruch 2, **dadurch gekennzeichnet, dass** der Behälter (12) eine Welle (56) mit einem auf einem Ende der Welle (56) gelagerten Magnetkörper (54) eines Mischorgans aufweist, der in eine Aufnahme (32) des Bodenteils (14) oder des Deckenteils (16) eingesetzt ist.

## Claims

1. A packaging (10) for a flexible container (12), in particular for a disposable bag for use in a bioreactor, comprising
a bottom part (14),
a ceiling part (16) having means for fixing a first portion (60) of the container (12),
a receiving space (20) which is defined on a first side by the bottom part (14) and on an opposite, second side by the ceiling part (16), and
struts (18) which are arranged between the bottom part (14) and the ceiling part (16) and fix a defined distance between the bottom part (14) and the ceiling part (16),
the bottom part (14) including means for fixing a second portion (58), opposite to the first portion (58), of the container (12), so that in a transport condition, the fixed flexible container (12) can be permanently held in an unfolded condition,
**characterized in that**
the bottom part (14), the ceiling part (16) and the struts (18) are manufactured from the same, gamma-sterilizable, material, preferably polyethylene (PE), and
that for each strut (18), the bottom part (14) and the ceiling part (16) include opposite fixtures (48) in which the struts (18) are inserted parallel to each other to form a packaging frame.

2. A transport unit comprising a disposable bag (12) for use in a bioreactor, **characterized in that** the disposable bag (12) is received in a packaging (10) according to claim 1, the first portion (60) of the disposable bag (12) being fixed to the ceiling part (16) and the opposite, second portion (58) of the disposable bag (12) being fixed to the bottom part (14), so that in a transport condition, the fixed disposable bag (12) is permanently held in an unfolded condition.

3. The transport unit according to claim 2, **characterized in that** the container (12) includes a shaft (56) having a magnetic body (54) of a mixing tool, the magnetic body being supported on an end of the shaft (56) and being inserted in a receptacle (32) of the bottom part (14) or of the ceiling part (16).

## Revendications

1. Emballage (10) pour un récipient souple (12), en particulier pour un sachet jetable pour l'utilisation dans un bioréacteur, comprenant
une pièce de fond (14),
une pièce de plafond (16) présentant des moyens pour la fixation d'un premier tronçon (60) du récipient (12),
un espace de logement (20) qui est délimité d'un premier côté par la pièce de fond (14) et d'un deuxième côté opposé par la pièce de plafond (16), et
des entretoises (18) qui sont agencées entre la pièce de fond (14) et la pièce de plafond (16) et qui déterminent une distance définie entre la pièce de fond (14) et la pièce de plafond (16),
la pièce de fond (14) présentant des moyens pour la fixation d'un deuxième tronçon (58) du récipient (12) qui est opposé au premier tronçon (58), de sorte que dans un état de transport, le récipient souple (12) fixé peut être maintenu de manière durable dans un état déployé,
**caractérisé en ce que**
la pièce de fond (14), la pièce de plafond (16) et les entretoises (18) sont fabriquées en la même matière apte à être stérilisée par rayonnement gamma, de préférence en polyéthylène (PE), et
**en ce que** la pièce de fond (14) et la pièce de plafond (16) présentent pour chaque entretoise (18) des attaches (48) opposées dans lesquelles les entretoises (18) sont insérées parallèles les unes aux autres pour former un cadre d'emballage.

2. Unité de transport, comportant un sachet jetable (12) pour l'utilisation dans un bioréacteur, **caractérisé en ce que** le sachet jetable (12) est reçu dans un emballage (10) selon la revendication 1, le premier tronçon (60) du sachet jetable (12) étant fixé à la pièce de plafond (16) et le deuxième tronçon opposé (58) du sachet jetable (12) étant fixé à la pièce de fond (14) de sorte que dans un état de transport, le sachet jetable (12) fixé est maintenu de manière durable dans un état déployé.

3. Unité de transport selon la revendication 2, **caractérisé en ce que** le récipient (12) comporte un arbre (56) qui présente un corps magnétique (54) d'un organe de mélange qui est monté sur une extrémité de l'arbre (56) et qui est inséré dans un logement (32) de la pièce de fond (14) ou de la pièce de plafond (16).
